# EUROPÄISCHE PATENTSCHRIFT

(11) **EP 1 173 542 B1**
(45) Veröffentlichungstag und Bekanntmachung des Hinweises auf die Patenterteilung: **20.04.2005**
(21) Anmeldenummer: 00918654.5
(22) Anmeldetag: 03.05.2000
(51) Int. Cl.: C12M 3/00

(54) **BIOREAKTOR**
BIOREACTOR
BIOREACTEUR

(30) Priorität: 04.05.1999 CH 82899
(43) Veröffentlichungstag der Anmeldung: 23.01.2002
(73) Patentinhaber: Röll, Marcel, 8124 Maur (CH)
(72) Erfinder: Röll, Marcel, 8124 Maur (CH)
(74) Vertreter: Schmauder, Klaus Dieter, Dipl.-Ing.
(86) Internationale Anmeldenummer: PCT/CH2000/000245
(87) Internationale Veröffentlichungsnummer: WO 2000/066706

(56) Entgegenhaltungen:
- EP-A- 0 258 795
- EP-A- 0 725 134
- WO-A-87/00548
- FR-A- 2 519 020
- US-A- 5 523 228
- PATENT ABSTRACTS OF JAPAN vol. 015, no. 123 (C-0816), 26. März 1991 (1991-03-26) & JP 03 007575 A (SHIMADZU CORP), 14. Januar 1991 (1991-01-14)
- PATENT ABSTRACTS OF JAPAN vol. 015, no. 128 (C-0818), 28. März 1991 (1991-03-28) & JP 03 010676 A (SHIMADZU CORP), 18. Januar 1991 (1991-01-18)

## Beschreibung

### Technisches Gebiet

Die Erfindung betrifft einen Bioreaktor gemäss Oberbegriff des Anspruches 1.

### Stand der Technik

Ein Bioreaktor der eingangs genannten Art ist aus der FR-A-2 519 020 (DE-A-32 48 543) bekannt. Der bekannte Bioreaktor ist nur für kleinere Fassungsvermögen, beispielsweise 2,5 Liter, beschrieben. Die horizontale Lage ist für die Reaktion des zu behandelnden Gutes förderlich, erschwert jedoch das Entleeren und insbesondere ein Trennen von Medium und Zellkultur.

Analoges gilt auch für die Bioreaktoren gemäss der EP-A-0 258 795 und der JP-A-03007575, wobei in ersterem Falle erschwerend hinzukommt, dass der Beutelreaktor mehrkammerig ausgebildet ist. Eine erste Kammer dient zur Aufnahme der Zellkultur und des Mediums und mindestens eine zweite Kammer dient zur Zuführung von Behandlungsgas. Die erste und die zweite Kammer sind durch eine gaspermeable Wand getrennt, die den Gasdurchtritt erschwert und damit die Reaktionen verzögert und im Falle einer Verschmutzung den Gasdurchtritt sogar unterbrechen kann. Bei dem Bioreaktor nach der JP-A-03007575 erweist sich die taumelnde Bewegung überdies als nachteilig, da sie eher zu einer Drehung des zu behandelnden Gutes als zu einer erforderlichen Durchmischung führt.

### Darstellung der Erfindung

Aufgabe der Erfindung ist es, den Bioreaktor der eingangs genannten Art zu verbessern.

Die Aufgabe wird gelöst durch die kennzeichnenden Merkmale des Anspruches 1.

Der Reaktorbeutel ermöglicht ein grösseres Fassungsvolumen, da die mit einem höheren Fassungsvermögen verbundenen höheren Belastungen durch die Anordnung der Schale in einem Schwingrahmen besser bewältigt werden können. Diese Ausgestaltung ermöglicht auch einen grösseren Schwenkwinkel sowie eine vertikale Lage, da der Schwingrahmen zwischen Seitenteilen des Reaktorgestelles angeordnet ist, die eine grössere Bewegungsfreiheit des Schwingrahmens ermöglichen.

Vorteilhafte Ausgestaltungen des Bioreaktors sind in den Ansprüchen 2 bis 8 beschrieben.

Zur Begrenzung des Schwenkwinkels können gemäss Anspruch 2 Anschläge am Reaktionsgestell vorhanden sein. Für den Antrieb des Schwingrahmens sind verschiedene Varianten möglich, wobei ein Antriebsrad gemäss Anspruch 3 mit einem Bogenglied des Schwingrahmens zusammenwirken kann, dessen Bogenmittelpunkt in der Schwenkachse liegt. Hierzu kann das Bogenglied beispielsweise eine Aussenverzahnung aufweisen und das Antriebsrad ein entsprechendes Ritzel. Eine besonders einfache Lösung des Antriebes beschreibt hingegen Anspruch 4.

Der Reaktorbeutel kann eine beliebige Form und beliebige Mittel zur Befestigung in der Schale aufweisen, es hat sich jedoch herausgestellt, dass eine Ausbildung nach Anspruch 5 besonders bevorzugt ist. Auch für die Ausgestaltung der Spannvorrichtung ergeben sich verschiedene Möglichkeiten, eine besonders einfache und sichere Befestigung gewährleistet die Ausgestaltung nach Anspruch 6. Mit der Weiterbildung nach Anspruch 7 ist ein sehr schnelles Öffnen der Spannvorrichtung und damit Auswechseln des Reaktorbeutels möglich. Durch die Weiterbildung nach Anspruch 8 kann die Spannkraft eingestellt und insbesondere die Spannvorrichtung schnell an Reaktorbeutel unterschiedlicher Dickenmasse angepasst werden.

### Kurze Beschreibung der Zeichnungen

Ausführungsbeispiele des Bioreaktors werden nachfolgend anhand der Zeichnungen näher beschrieben, dabei zeigen:
- Figur 1: einen ersten Bioreaktor in Ansicht auf die Schmalseite;
- Figur 2: den Bioreaktor der Figur 1 im Schnitt II-II der Figur 1;
- Figur 3: einen Reaktorbeutel des Bioreaktors der Figur 1 in schaubildlicher Darstellung;
- Figur 4: eine Einspannvorrichtung für den Reaktorbeutel in grösserem Massstab;
- Figur 5: einen zweiten Bioreaktor in Ansicht auf die Schmalseite und teilweise geschnitten.

### Wege zur Ausführung der Erfindung

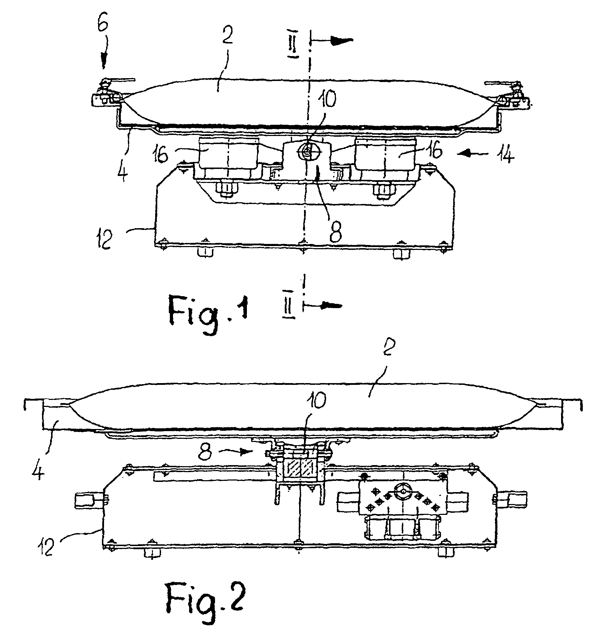

Die Figuren 1 bis 4 zeigen einen ersten Bioreaktor mit einer bevorzugten Befestigung eines Beutelreaktor 2 in einer Schale 4. Der Beutelreaktor 2 ist an gegenüberliegenden Seiten mittels einer Einspannvorrichtung 6 gehalten. Diese Schale 4 ist über ein Lager 8 um eine Schwingachse 10 schwingbar an einem Reaktorgestell 12 gelagert. Ein Schwingantrieb 14 versetzt die Schale 4 in auf- und abgehende Schwingungen. Der Schwingantrieb wird in nicht näher dargestellter Weise mittels eines Fluid, vorzugsweise Druckluft, angetrieben und enthält zwei beidseits des Lagers 8 angeordnete Hubzylinder 16, die zwischen der Schale 4 und dem Reaktorgestell 12 angeordnet sind.

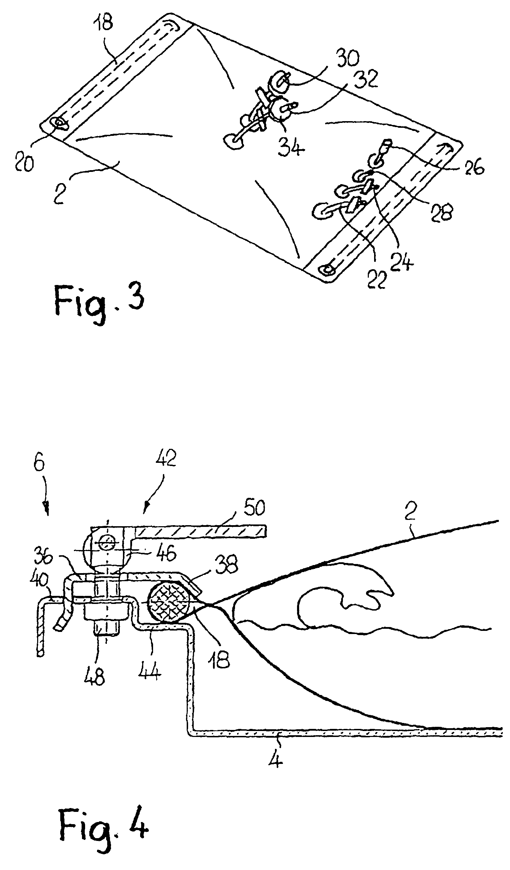

Der Reaktorbeutel 2 ist aus Kunststoff ausgebildet und enthält, wie aus Figur 3 hervorgeht, an den gegenüberliegenden Schmalseiten Säume 18, die als Hohlsäume ausgebildet sind und in denen ein Keder 20 beispielsweise in Form eines Rundstabes aus Kunststoff angeordnet ist. Der Beutelreaktor 2 ist mit einem Einfüllanschluss 22 und einem Entleeranschluss 24 für das zu behandelnde Gut ausgerüstet. Weiter enthält der Reaktorbeutel einen Probeentnahmeanschluss 26 und einen Reserveanschluss 28. Schliesslich ist auf der Oberseite des Beutelreaktors ein Zuführanschluss 30 und ein Abführanschluss 32 für Behandlungsgas vorhanden, in denen jeweils ein Filter 34 angeordnet ist.

Der Beutelreaktor liegt in der Schale 4 des Bioreaktors und ist an seinen Schmalseiten mittels der erwähnten Einspannvorrichtungen 6 in der Schale befestigt. Jede Halteschale weist eine Halteleiste 36 auf, welche einen den Saum 18 mit dem Keder 20 übergreifenden Rückhalteflansch 38 enthält. Dieser wird mittels eines mit dem Rand 40 der Schale 4 zusammenwirkenden Niederhalters 42 gegen einen den Saum 18 unterliegenden Auflagerand 44 der Schale 4 gepresst. Der Niederhalter 42 weist hierzu einen Exzenter 46 auf, der an einem mit dem Rand 40 verschraubten Bolzen 48 drehbar gelagert ist und mittels eines Handhebels 50 gegen die Halteleiste gepresst wird.

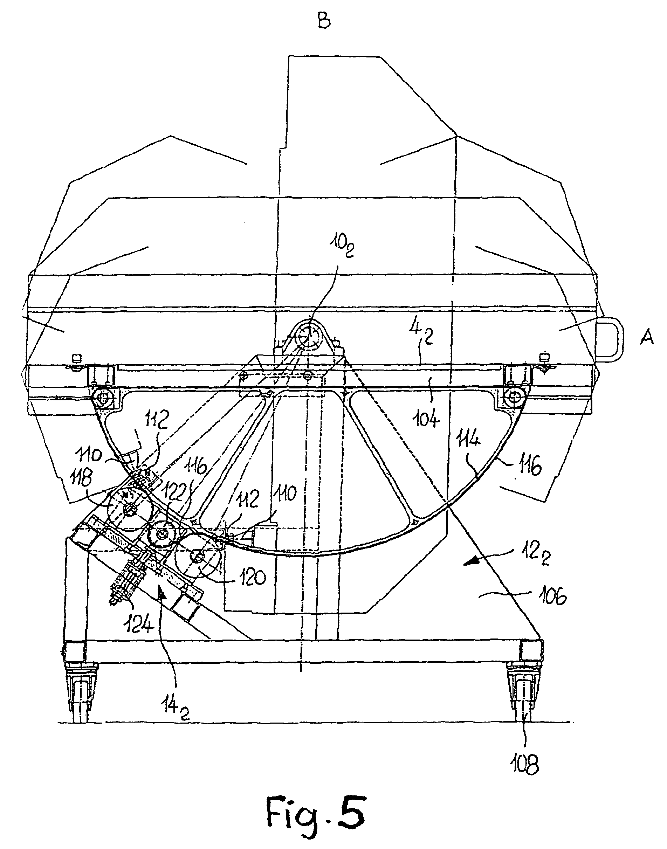

Die Figur 5 zeigt einen zweiten Bioreaktor, dessen Schale 4₂ in einem Schwingrahmen 104 angeordnet ist, der über die Schwingachse 10₂ mit Seitenteilen 106 des Reaktorgestells 12₂ verschwenkbar verbunden ist. Wie aus Figur 5 zu entnehmen ist, ist das Reaktorgestell mittels Rädern 108 verfahrbar ausgebildet. Zur Begrenzung des Schwenkwinkels sind am Schwingrahmen gegebenenfalls einstellbare Anschläge 110 in Form von Gummipuffern angeordnet, die mit Anschlägen 112 am Reaktorgestell 12₂ zusammenwirken. Der Schwingantrieb 14₂ wird gebildet durch ein Bogenglied 114, das mit dem Schwingrahmen 104 verbunden ist und dessen Bogenmittelpunkt mit der Schwingachse 10₂ zusammenfällt. Am Bogenglied 114 ist eine Antriebskette oder ein Antriebsriemen 116 aufgespannt, der über Umlenkrollen 118,120 gegen ein Antriebsrad 122 umgelenkt ist und dieses umschlingt. Das Antriebsrad ist mittels eines nicht näher dargestellten Motors und einem entsprechenden Getriebe antreibbar. Eine Spannvorrichtung 124 dient zum Spannen der Antriebskette beziehungsweise des Antriebsriemens.

Dieser Bioreaktor ermöglicht einen sehr grossen Schwenkwinkel bis zu 110°, wie dies in Figur 5 angedeutet ist und kann durch entsprechende Steuerung des Schwingantriebes verändert werden. Im Reaktorbetrieb (Pos. A) ist der Schwingrahmen 104 beispielsweise in einem Schwingwinkel von 6° bis 16° um die horizontale Achse schwingbar, wie dies in Figur 5 angedeutet ist. Der Schwingrahmen 104 kann aber auch in eine senkrechte Position B verschwenkt werden, in der sich beispielsweise Zellkulturen eines Reaktionsgemisches unten absetzen und ein Medium wie eine Nährlösung sich darüber schichtet. Das Medium kann dann abgesaugt und die Zellkultur gewonnen oder das Medium kann ausgetauscht werden.

Während der Bioreaktor der Figuren 1 bis 4 ein Fassungsvermögen des Reaktorbeutels von 1 bis 20 Liter aufweisen kann, ermöglicht der Bioreaktor gemäss der Figur 5 ein wesentlich grösseres Fassungsvermögen des Reaktorbeutels. Ein solches Fassungsvermögen kann beispielsweise 200 Liter betragen.

### BEZUGSZEICHENLISTE

- 2: Beutelreaktor
- 4: Schale
- 4₂: Schale
- 6: Einspannvorrichtung
- 8: Lager
- 10: Schwingachse
- 10₂: Schwingachse
- 12: Reaktorgestell
- 12₂: Reaktorgestell
- 14: Schwingantrieb
- 14₂: Schwingantrieb
- 16: Hubzylinder
- 18: Saum
- 20: Keder
- 22: Einfüllanschluss
- 24: Entleeranschluss
- 26: Probeentnahmeanschluss
- 28: Reserveanschluss
- 30: Zuführanschluss
- 32: Abführanschluss
- 34: Filter
- 36: Halteleiste
- 38: Rückhalteflansch
- 40: Rand
- 42: Niederhalter
- 44: Auflagerand
- 46: Exzenter
- 48: Bolzen
- 50: Handhebel
- 104: Schwingrahmen
- 106: Seitenteile
- 108: Rad
- 110: Anschlag
- 112: Anschlag
- 114: Bogenglied
- 116: Antriebskette/riemen
- 118: Umlenkrolle
- 120: Umlenkrolle
- 122: Antriebsrad
- 124: Spannvorrichtung

## Patentansprüche

1. Bioreaktor mit einem als Kunststoffbeutel ausgebildeten einkammerigen Reaktorbehälter mit Zu- und Ableitungen (22,24,26,28,30,32), wobei dieser Beutelreaktor (2) an gegenüberliegenden Seiten in einer Schale (4,4₂) mittels einer Einspannvorrichtung (6) gehalten ist, wobei die Schale mittels eines Schwingantriebes (14,14₂) um eine -horizontale Schwingachse (10,10₂) eines Reaktorgestelles (12,12₂) schwingbar ist, wobei er Mittel (56,54,112,114,116,122) zur Einstellung der Grösse des Schwenkwinkels aufweist, **dadurch gekennzeichnet, dass** die Schale (4₂) in einem Schwingrahmen (104) angeordnet ist, der zwischen Seitenteilen (106) des Reaktorgestells (12₂) um die Schwingachse (10₂) schwingbar ist, und mit dem der Schwingantrieb (14₂) verbunden ist, sodass die Schale (4₂) aus der horizontalen Lage in eine vertikale Lage verschwenkbar ist.

2. Bioreaktor nach Anspruch 1, **dadurch gekennzeichnet, dass** am Reaktorgestell (12₂) Anschläge (112) zur Begrenzung des Schwenkwinkels des Schwingrahmens (104) angeordnet sind.

3. Bioreaktor nach Anspruch 1 oder 2, **dadurch gekennzeichnet, dass** der Schwingantrieb (14₂) ein Antriebsrad (122) aufweist, das mit einem Bogenglied (114) des Schwingrahmens (104) zusammenwirkt, dessen Bogenmittelpunkt in der Schwingachse (10₂) liegt.

4. Bioreaktor nach Anspruch 3, **dadurch gekennzeichnet, dass** das Antriebsrad (122) mit einer Antriebskette (116) oder einem Antriebsriemen zusammenwirkt, die/der am Umfang des Bogengliedes (114) angeordnet ist.

5. Bioreaktor nach einem der Ansprüche 1 bis 4, **dadurch gekennzeichnet, dass** der Beutelreaktor (2) rechteckig ausgebildet ist und an gegenüberliegenden Seiten, vorzugsweise den Schmalseiten, jeweils einen hohlen Saum (18) mit einem Keder (20) aufweist, so dass der Saum (18) in der Einspannvorrichtung (6) der Schale (4) einspannbar ist.

6. Bioreaktor nach Anspruch 5, **dadurch gekennzeichnet, dass** die Einspannvorrichtung (6) eine Halteleiste (36) aufweist, welche einen den Saum (18) mit dem Keder (20) übergreifenden Rückhalteflansch (38) enthält und mittels eines mit dem Rand (40) der Schale (4) zusammenwirkenden Niederhalters (42) gegen einen den Saum (18) unterliegenden Auflagerand (44) der Schale (4) pressbar ist.

7. Bioreaktor nach Anspruch 6, **dadurch gekennzeichnet, dass** der Niederhalter (42) einen mittels eines Handhebels (50) verschwenkbaren Exzenter (46) aufweist, der an der Halteleiste (36) anliegt.

8. Bioreaktor nach Anspruch 7, **dadurch gekennzeichnet, dass** der Exzenter (46) in einem mit dem Rand (40) der Schale (4) verschraubbaren Bolzen (48) gelagert ist.

## Claims

1. Bioreactor having a single-chamber reactor container configured as a plastic bag with inlets and outlets (22, 24, 26, 28, 30, 32), this bag reactor (2) being held on opposite sides in a basin (4, 4₂) by means of a clamping device (6), the basin being capable of being made to oscillate about a horizontal axis of oscillation (10, 10₂) of a reactor frame (12, 12₂) by means of an oscillating drive (14, 14₂), said reactor comprising means (56, 54, 112, 114, 116, 122) for adjusting the magnitude of the pivot angle, **characterised in that** the basin (4₂) is arranged in an oscillating frame (104) which is capable of oscillating about the axis of oscillation (10₂) between side parts (106) of the reactor frame (12₂), and to which the oscillating drive (14₂) is connected, so that the basin (4₂) can be pivoted out of the horizontal position into a vertical position.

2. Bioreactor according to claim 1, **characterised in that** stops (112) for limiting the pivot angle of the oscillating frame (104) are provided on the reactor frame (12₂).

3. Bioreactor according to claim 1 or 2, **characterised in that** the oscillating drive (14₂) comprises a drive wheel (122) which co-operates with an arc member (114) of the oscillating frame (104), the centre of the arc thereof being located on the axis of oscillation (10₂).

4. Bioreactor according to claim 3, **characterised in that** the drive wheel (122) co-operates with a drive chain (116) or a drive belt which is arranged on the circumference of the arc member (114).

5. Bioreactor according to one of claims 1 to 4, **characterised in that** the bag reactor (2) is of rectangular construction and comprises on opposite sides, preferably the short sides, a hollow seam (18) with piping (20), so that the seam (18) can be secured in the clamping device (6) of the basin (4).

6. Bioreactor according to claim 5, **characterised in that** the clamping device (6) comprises a holding strip (36) which contains a retaining flange (38) overlapping the seam (18) with the piping (20) and is adapted to be pressed against a support edge (44) of the basin (4) underneath the seam (18) by means of a pressing member (42) co-operating with the edge (40) of the basin (4).

7. Bioreactor according to claim 6, **characterised in that** the pressing member (42) comprises an eccentric (46) which is pivotable by means of a hand-operated lever (50) and which bears on the holding strip (36).

8. Bioreactor according to claim 7, **characterised in that** the eccentric (46) is mounted in a bolt (48) which can be screwed to the edge (40) of the basin (4).

## Revendications

1. Bioréacteur comportant un récipient de réaction à chambre unique réalisé sous la forme d'un sac en matière plastique muni de conduites d'entrée et de sortie (22, 24, 26, 28, 30, 32), ce réacteur du type sac (2) étant retenu sur des côtés se faisant face dans une coque (4, 4₂) au moyen d'un dispositif de fixation (6), la coque vibrant au moyen d'un mécanisme de vibration (14, 14₂) autour d'un axe de pivotement horizontal (10, 10₂) d'un châssis de réacteur (12, 12₂), celui-ci présentant des moyens (56, 54, 112, 114, 116, 122) pour régler l'amplitude de l'angle de pivotement,
**caractérisé en ce que**
la coque (4₂) est disposée dans un bâti vibrant (104), qui peut vibrer entre des parties latérales (106) du châssis de réacteur (12₂) autour de l'axe de pivotement (10₂), et auquel le mécanisme de vibration (14₂) est relié, de sorte que la coque (4₂) puisse pivoter de la position horizontale à la position verticale.

2. Bioréacteur selon la revendication 1,
**caractérisé en ce que**
sur le châssis de réacteur (12₂) sont disposées des butées (112) pour limiter l'angle de pivotement du bâti vibrant (104).

3. Bioréacteur selon la revendication 1 ou 2,
**caractérisé en ce que**
le mécanisme de vibration (14₂) présente une roue motrice (122), qui coopère avec un guide courbé (114) du bâti vibrant (104) dont le centre de courbure se trouve dans l'axe de pivotement (10₂).

4. Bioréacteur selon la revendication 3,
**caractérisé en ce que**
la roue motrice (122) coopère avec une chaîne motrice (116) ou une courroie de transmission, qui est disposée à la périphérie du guide courbé (114).

5. Bioréacteur selon l'une quelconque des revendications 1 à 4,
**caractérisé en ce que**
le réacteur du type sac (2) est de forme rectangulaire et présente sur des côtés se faisant face, de préférence, des côtés étroits, respectivement un ourlet creux (18) avec un bourrelet (20), de sorte que l'ourlet (18) puisse être fixé dans le dispositif de fixation (6) de la coque (4).

6. Bioréacteur selon la revendication 5,
**caractérisé en ce que**
le dispositif de fixation (6) présente une barrette de maintien (36), qui contient une bride de retenue (38) agrippant l'ourlet (18) avec le bourrelet (20) et peut être pressée au moyen d'un serre-flan (42) coopérant avec le bord (40) de la coque (4) contre un bord d'appui (44) de la coque (4) plaçant l'ourlet (18) en dessous.

7. Bioréacteur selon la revendication 5,
**caractérisé en ce que**
le serre-flan (42) présente un excentrique (46) pouvant être pivoté au moyen d'un levier manuel (50), qui prend appui contre la barrette de maintien (36).

8. Bioréacteur selon la revendication 7,
**caractérisé en ce que**
l'excentrique (46) est placé dans un boulon (48) pouvant être vissé avec le bord (40) de la coque (4).
